# EUROPÄISCHE PATENTSCHRIFT

(11) **EP 1 565 567 B1**
(45) Veröffentlichungstag und Bekanntmachung des Hinweises auf die Patenterteilung: **24.02.2010**
(21) Anmeldenummer: 03779963.2
(22) Anmeldetag: 17.11.2003
(51) Int. Cl.: C12Q 1/02, C07K 16/30, C07K 16/28

(54) **VERFAHREN ZUR SELEKTION VON ZELLEN, DIE SPEZIFISCH BINDENDE MOLEKÜLE PRODUZIEREN**
METHOD FOR THE SELECTION OF CELLS PRODUCING SPECIFICALLY BINDING MOLECULES
PROCEDE DE SELECTION DE CELLULES PRODUISANT DES MOLECULES A LIAISON SPECIFIQUE

(30) Priorität: 30.11.2002 DE 10256042
(43) Veröffentlichungstag der Anmeldung: 24.08.2005
(73) Patentinhaber: Universität Potsdam, 14469 Potsdam (DE)
(72) Erfinder: SELLRIE, Frank, 12559 Berlin (DE); MICHEEL, Burkhard, 14476 Golm (DE)
(74) Vertreter: Krauss, Jan
(86) Internationale Anmeldenummer: PCT/EP2003/012863
(87) Internationale Veröffentlichungsnummer: WO 2004/050901

(56) Entgegenhaltungen:
- EP-A- 0 974 649
- WO-A-01/90153
- WO-A-94/02016
- WO-A-95/24220
- DE-A- 10 038 573
- DE-A- 10 060 959
- US-A- 5 352 595
- US-A- 5 411 861
- US-A- 5 658 568
- SZARDENINGS MICHAEL ET AL: "Phage display selection on whole cells yields a peptide specific for melanocortin receptor 1" JOURNAL OF BIOLOGICAL CHEMISTRY, AMERICAN SOCIETY OF BIOLOGICAL CHEMISTS, BALTIMORE, MD, US, Bd. 272, Nr. 44, 31. Oktober 1997 (1997-10-31), Seiten 27943-27948, XP002193667 ISSN: 0021-9258

## Beschreibung

Die vorliegende Erfindung betrifft ein Verfahren zur Selektion von Zellen, die spezifisch bindende Moleküle exprimieren, dessen Anwendung und die Verwendung eines erfindungsgemäßen Kits. Das Verfahren kann auf alle Zellen angewandt werden, die in der Lage sind, spezifisch bindende Moleküle zu exprimieren, wie zum Beispiel Bakterienzellen, Hefezellen, Pilzzellen, Insektenzellen, Algenzellen, Pflanzenzellen und Säugerzellen und insbesondere Hybridomzellen und Stammzellen, wie etwa nicht-menschlichen embryonalen Stammzellen. Das Verfahren erlaubt auch eine einfache Selektion, bei der die Zellen in Form eines Organs und/oder Gewebes vorliegen. Zusätzlich kann die Selektion in vitro oder in vivo durchgeführt werden. Als zu exprimierende Moleküle können Nukleinsäuren, Polysaccharide oder Proteine und insbesondere Antikörper und Antikörperfragmente selektiert werden.

Viele der Ansätze in der modernen molekularen Medizin beruhen auf der Auffindung von spezifischen Interaktionen zwischen zwei Bindungspartnern, üblicherweise als Rezeptor und Ligand/Binder oder Liganden bezeichnet. So werden in teilweise sehr aufwendigen und teuren Verfahren z. B. die Interaktionen zwischen zellulären Proteinen untereinander, Antikörper-Antigen-Bindungen, Nukleinsäure-Interaktionen und die Interaktionen von Rezeptoren der Zellen mit einer Vielzahl von extrazellulären Faktoren in zum Beispiel so genannten "Compound-Libraries" (Substanzbibliotheken) untersucht.

Von besonderer Wichtigkeit sind die Selektion von spezifischen Antikörpern oder Antikörperfragmenten, die z. B. in der Medizin, Umwelttechnik, Diagnostik etc., eingesetzt werden können und die Selektion von spezifischen Liganden in der Pharmabranche für Wirkstoffentwicklung, die dann ebenfalls in der Medizin zum Einsatz kommen können. So können die aufgefundenen Interaktionen zum einen zur Diagnostik, z. B. in der Diagnostik von bestimmten Erkrankungen, verwendet werden, zum anderen aber auch zur Identifizierung von "Targets" für die Wirkstoffforschung, bei denen dann der bindende Ligand als Ausgangsstruktur ("core-structure") für einen Wirkstoff und somit zur Therapie dienen kann. Weiterhin kann die Erfindung natürlich auch einem erweiterten Verständnis zellulärer Prozesse und biologischer Funktionen dienen. Besondere Beispiele hierfür sind die Untersuchung der Apoptose und die Wirkung von Hormonen oder anderen Botenstoffen des Körpers.

Grundsätzlich benötigt man für alle der oben genannten Untersuchungsverfahren eine Zelle, die einen Rezeptor oder Liganden, im folgenden allgemein als "Molekül" oder "bindendes Molekül" bezeichnet, exprimiert. Dieses Molekül kann insbesondere ein Makromolekül in Form z.B. eines Proteins oder Peptids sein. Bisher bediente man sich zum Auffinden bindender Moleküle im wesentlichen verschiedener Sceeningmethoden wie z. B. dem Enzymimmunassay (ELISA) bei der Hybridomtechnik oder des "Pannings" beim "Phage display", wie unter anderem in den Publikationen von Köhler und Milstein (C. Köhler G, Milstein C. Continuous cultures of fused cells Secreting antibody of predefined specificity. Nature. 1971 Aug 7:256(5517):495-7.), Clackson et al. (Clackson T, Hoogenboom HR, Griffiths AD, Winter G. Making antibody fragments using phage display Iibraries. Nature. 199x Aug 15;352(6336):624-8.) und Hanes und Plückthun (Hanes J, Plückthun A. In vitro selection and evolution of functional proteins by using rtbosome display. 6 Proc NatlAcad Sci USA. 1997 May 13:94(10):4937-42.) beschrieben. Weiterhin soll das "Two-Hybrid-System" zur Selektion von Protein-Protein-Bindern erwähnt werden (Fields S, Song O.; A novel genetic system to detect protein-protein interactions. Nature. 1989, 340 (6230):245-246).

Screeningmethoden sind zeit- und materialaufwendig und damit teuer, da jedes Einzelereignis durch geeignete Tests separat bewertet werden muß. Dadurch muß in jedem Falle die Gesamtmenge an Ereignissen auf eine Menge eingeschränkt werden, aus denen noch eine Auswahl erfolgen kann. Die Suche nach seltenen Ereignissen wird erschwert bzw. unmöglich.

Je nach zu untersuchender Fragestellung, wie zum Beispiel im Falle von verschiedenen Zelltypen oder -quellen, erfordern die verschiedenartigen Ansätze der Screeningverfahren des Stands der Technik immer ein jeweils genau auf die Untersuchung ausgerichtetes System. Dies begrenzt die Zahl der möglichen Ergebnisse noch weiter. Ein einfaches und universell einsetzbares System zur Selektion von exprimierenden Zellen existiert bisher nicht.

Der Stand der Technik versucht das oben genannte Problem mit einer Automatisierung des Screenings zu umgehen, was die beschriebenen Probleme zwar mindert, sie jedoch nicht löst. Halbautomatische Isolationsverfahren existieren für das Auffinden von Antikörperfragmenten aus Phagenbibliotheken (Phage display, siehe oben). Einer weitgehenden Automatisierung des Screenings von lebendigen Zellen, wie zum Beispiel Hybridomen oder Stammzellen stehen im wesentlichen Sterilitätsprobleme im Weg.

EP 0 974 649 offenbart ein Screening-System zum Screening von Effektorpeptiden (spezifisch bindenden Molekülen) als Agonisten oder Antagonisten für Zielproteine oder - proteinkomplexe, wie ein p53-Tetramer oder einen Komplex aus einer Bindungsdomäne und einer Aktivierungsdomäne (Bsp. VEGF). Dabei werden eukaryotische Wirtszellen, die ein Selektionssystem meist ein "monitoring"-Gen) umfassen, mit einer DNA-Bibliothek transformiert, die für die Effektorpeptide kodiert und danach auf Überleben der Zellen selektioniert. Dies ist möglich, weil in Abhängigkeit von der Bindung der Effektorpeptide das Zielprotein/-komplex mit einem weiteren regulatorischen Protein interagieren und darüber das "monitoring"-Gen angeschaltet werden kann.

US 5,658,568 offenbart ein therapeutisches System zur Zerstörung von Zielzellen (wie Krebszellen) in einem Wirt. Dabei wird die Wirkung eines zytotoxischen Mittels am gewünschten Wirkort (dem Tumor) dadurch verlängert, daß dem Wirt gleichzeitig zum zytotoxischen Mittel ein Inhibitor desselben sowie ein Konjugat aus Krebszell-spezifischer Bindungsdomäne und einer den Inhibitor inaktivierenden Domäne (wie ein Antikörper-Enzym-Konjugat) verabreicht wird. Dabei findet eine Art Selektion der zu zerstörenden Zielzellen statt, und zwar dadurch, daß das Konjugat spezifisch an die Ziel-Krebszellen bindet.

US 5,411,861 beschreibt ein Verfahren zum Mapping von Epitopen mittels Mutationsanalyse. Im dargestellten Verfahren wird die Reaktivität von Bindedomänen, insbesondere Antigen-Antikörper Bindungen, dadurch festgestellt, dass die zu selektierenden Proteinbindungsdomänen auf der Oberfläche von Zellen exprimiert werden und zuerst einer ersten negativen Selektion mit einem ersten Selektionsmittel (einem ersten Antikörper) und danach einer zweiten positiven Selektion mit einem zweiten Selektionsmittel (einem zweiten Antikörper) unterzogen werden.

In der Oßenteeunesschrift der deutschen Patentanmeldung DE 100 60 959 beschreiben die Erfinder ein Verfahren zur optimierten HTS (High Throughput Screening) mittels einer Bibliothek von Komponenten, wobei jeder einzelnen Substanz der Komponentenbibliothek eine Nukleinsäure angehängt ist. In einem Screening kann diese für jede Komponente spezifische Nukleinsäuresectuenz zur Identifikation des Kandidaten Wirkstoffs herangezogen werden.

In der WO 01/90153 werden Peptid-Ligandenkoniugate beschrieben, mit denen spezifische Populationen von Zellen ausgewählt werden sollen. Die Zellspezifität des Systems wird über den Liganden des Peptid-Ligandenkonjugats bereit gestellt, der beispielsweise ein Zytokin oder auch ein Antikörper sein kann. Der Peptidanteil des Konjugats wird wiederum durch einen zweiten Liganden (z.B. Antikörper) gebunden. Ist der zweite Ligand fest an eine Matrix gebunden, können so gezielt Zellpopulationen aufgereinigt werden. Durch Zugabe eines freien (nicht konjugierten) Peptids wird die Bindung zwischen dem zweiten Liganden und dem Peptid-Ligandenkonjugat kompetitiv gelöst und die aufgereinigten Zellen somit freigesetzt.

Im Hinblick auf das oben Genannte ist es eine Aufgabe der Erfindung, ein Verfahren zur Selektion von Zellen zur Verfügung zu stellen, die spezifisch bindende Moleküle exprimieren/produzieren. Bei den zu selektierenden Bindern kann es sich um alle Arten von Molekülen (z.B. Nukleinsäuren, Polysacchariden, Proteinen und Peptiden) handeln, die von Zellen produziert werden können. Von besonderem Wert sollte die Erfindung für die Selektion von Proteinen und hier insbesondere für die Selektion von Antikörpern und Antikörperfragmenten sein. Ziel ist es letztendlich, einen im Vergleich zu den bisherigen Methoden vereinfachten Zugriff auf derartige bindende Moleküle zu erhalten. Weiterhin sollte das Verfahren auch zu dem sogenannten "Tissue Engineering" oder zur Selektion von transgenen Tieren und Pflanzen anwendbar sein.

Gemäß einem ersten Aspekt der vorliegenden Erfindung wird ein Verfahren zur Selektion von Zellen, die spezifisch bindende Moleküle exprimieren, zur Verfügung gestellt. Das Verfahren umfaßt die Schritte von: a) zur Verfügung stellen von Zellen, die potentiell mindestens ein spezifisch bindendes Molekül exprimieren, b) in Kontakt bringen der Zellen aus Schritt a) mit einem Konjugat, umfassend mindestens i) ein spezifisches Effektormolekül, das spezifisch auf das verwendete Zellsystem wirkt, und ii) einen spezifischen Liganden für das mindestens eine spezifisch bindende Molekül, und c) Selektion der Zellen, die das mindestens eine spezifisch bindende Molekül exprimieren.

Ein "Effektorrüolekül" oder "Effektorkomponente" im Sinne der vorliegenden Erfindung ist eine Substanz, das bzw. die den Stoffwechsel einer Zelle spezifisch so moduliert, daß der Einfluß für eine einfache Selektion nutzbar ist. Die Effektormoleküle können dabei unterschiedlichste Einflüsse auf die Zellen ausüben. Ein Effektormolekül kann unter bestimmten Bedingungen als Toxin zum Absterben einer Zelle führen, unter anderen Bedingungen über eine Suppressor/Aktivator-Wirkung im Hinblick auf eine Stoffwechselleistung der Zelle zu einer Wachstumsveränderung der Zelle führen, eine Farbänderung bewirken oder ähnliches.

Bisher bedient man sich zum Auffinden bindender Moleküle im wesentlichen verschiedener und komplizierter Screeningmethoden wie z.B. eines Enzymimmunassays (ELISA) bei der Hybridomtechnik oder eines Pannings beim Phage display. Ein einfaches und universell einsetzbares System zur Selektion existiert bisher nicht. Das bisher übliche Screening wird nun ersetzt durch das erfindungsgemäße Selektionsverfahren, das sich in bestimmten Ausführungsformen sogar auf einen einzigen Selektionsschritt reduzieren läßt. Nur Ereignisse mit gewünschter Eigenschaft (d.h. Zellen welche ein geeignetes Molekül/Binder produzieren) bleiben nach dem erfindungsgemäßen Selektionsschritt übrig und können einer anschließenden Charakterisierung zugeführt werden.

Das erfindungsgemäße Verfahren ist dadurch weniger zeit- und materialaufwendig - und damit billiger. Es ermöglicht zudem den einfachen Zugriff auf sehr seltene Ereignisse, da aus einer sehr großen Gesamtmenge ausgewählt (selektiert) werden kann. Das wesentliche Neue des Verfahrens besteht in der Kombination von Bindung einer Zielstruktur (eines Liganden) und dadurch bewirkte Effekte, wie zum Beispiel Stoffwechselveränderungen in der Zelle oder ein Überleben der Zelle, die das bindende Molekül produziert. Zellen, die ein derartiges Molekül nicht produzieren sterben bzw. können nicht wachsen oder anhand geänderter Phänotypen selektiert werden. Neben der negativen Selektion kann das erfindungsgemäße Verfahren auch im Rahmen einer positiven Selektion angewendet werden. So besteht die Möglichkeit, auf andere Signale, als der Unterscheidung zwischen lebenden/wachsenden und toten/ruhenden Zellen zu selektionieren, in dem ein Effektormolekül-Konjugat verwendet wird, in dem z.B. ein Zelltyp farbig wird durch die Wechselwirkung mit dem bindenden Konjugat. Eine denkbare Wirkungskette wäre dabei ein Konjugat aus Suppressor/Aktivator-Ligand, das an das Molekül bindet. Dadurch bleibt das Konjugat inaktiv, eine intrazelluläre Stoffwechselveränderung erfolgt nicht, ein Reportergen wird nicht exprimiert oder umgekehrt wodurch sich der Phänotyp der Zelle ändert (z.B. Leuchten der Zellen durch GFP-Expression oder Farbänderung der Zellen durch lacZ-Expression). Eine weitere Möglichkeit wäre die Ergänzung des Two-Hybrid-Systems durch Ligand-gekoppelte Transskriptionsfaktoren.

Bevorzugt ist weiterhin ein Verfahren gemäß der vorliegenden Erfindung, bei dem Schritt b) die Synthese eines Konjugats umfaßt. Diese Synthese beinhaltet die Schritte von: b') Synthetisieren eines Konjugats aus mindestens einer Effektormolekül-Komponente und dem mindestens einen spezifischen Liganden für das mindestens eine spezifisch bindende Molekül, b") Überprüfen der Aktivität (z.B. Erlangen eines Selektionsvorteils) des Konjugats an Zellen, die keinen spezifischen Liganden exprimieren und Ermitteln geeigneter Selektionsbedingungen, und b"') in Kontakt bringen der Zellen aus Schritt a) wie oben genannt mit einer geeigneten Konzentration des Konjugats.

Dieses erfindungsgemäße Verfahren umfaßt somit im ersten Schritt die Synthese eines Konjugats aus einem für die jeweiligen Zellen, Gewebe oder Organismen geeigneten Effektormoleküls, wie etwa einem Toxin und/oder Suppressor/Aktivator und dem Liganden (Zielstruktur, gegen die ein bindendes Molekül gesucht wird). Grundlage dieses Verfahrens der Erfindung ist ein Konjugat aus einer für den Produzenten des bindenden Moleküls effektiven Komponente, wie etwa einer toxischen Komponente (Toxin) und/oder Suppressor/Aktivator und einer zweiten Komponente (Ligand), gegen den ein Binder selektiert werden soll. Erfindungsgemäß können das Effektormolekül und der Ligand des Konjugats kovalent oder nicht kovalent miteinander verbunden sein. Solche Arten von Konjugaten und deren Herstellung sind dem Fachmann gut bekannt. Erfindungsgemäß kann das Konjugat optional weiterhin einen kovalenten oder nicht kovalenten Linker zwischen dem Effektormolekül und dem Liganden aufweisen. Solche Linker sind bei der Synthese von Konjugaten übliche Bestandteile und können zum Beispiel Peptidlinker sein. Die Herstellung und Synthese solcher Linker ist dem Fachmann ebenfalls gut bekannt. Beispielhafte Konjugate sind beschrieben in unter anderem Kubo T, Dubey K, Fujii M. A novel approach for the solid phase synthesis of DNA-peptide conjugates. Nucleosides Nucleotides Nucleic Acids. 2001 Apr-Jul;20(4-7):1321-4; Suzawa T, Nagamura S, Saito H, Ohta S, Hanai N, Kanazawa J, Okabe M, Yamasaki M. Enhanced tumor cell selectivity of adriamycin-monoclonal antibody conjugate via a poly(ethylene glycol)-based cleavable linker. J Control Release. 2002 Feb 19;79(1-3):229-42; Kubo T, Dubey K, Fujii M. A novel approach for the solid phase synthesis of DNA-peptide conjugates. Nucleosides Nucleotides Nucleic Acids. 2001 Apr-Jul;20(4-7):1321-4; Achilefu S, Jimenez HN, Dorshow RB, Bugaj JE, Webb EG, Wilhelm RR, Rajagopalan R, Johler J, Erion JL. Synthesis, in vitro receptor binding, and in vivo evaluation of fluorescein and carbocyanine peptidebased optical contrast agents; J Med Chem. 2002 May 9;45(10):2003-15; Jones DS, Coutts SM, Gamino CA, Iverson GM, Linnik MD, Randow ME, Ton-Nu HT, Victoria EJ. Multivalent thioether-peptide conjugates: B cell tolerance of an anti-peptide immune response. Bioconjug Chem. 1999 May-Jun;10(3):480-8; Verheijen JC, van der Marel GA, van Boom JH, Bayly SF, Player MR, Torrence PF. 2,5-oligoadenylate-peptide nucleic acids (2-5A-PNAs) activate RNase L. Bioorg Med Chem. 1999 Mar;7(3):449-55; Schaffer DV, Lauffenburger DA. Optimization of cell surface binding enhances efficiency and specificity of molecular conjugate gene delivery. J Biol Chem. 1998 Oct 23;273(43):28004-9 und Mehvar R. Dextrans for targeted and sustained delivery of therapeutic and imaging agents. J Control Release. 2000 Oct 3;69(1):1-25.

Wenn das Effektormolekül eine toxische Komponente ist, kann diese toxische Komponente des erfindungsgemäßen Konjugats jede für das jeweilige zelluläre System toxische Substanz sein, die das Wachstum inhibiert und/oder die Zellen oder Gewebe abtötet. Erfindungsgemäß bevorzugt ist ein Verfahren, wobei die toxische Komponente ausgewählt ist aus Toxinen, wie etwa bakteriellen Toxinen. Toxine sind im Stand der Technik gut bekannt und umfassen unter anderem Escherichia coli Hitze-labiles Enterotoxin, *Escherichia coli* Hitzestabiles Enterotoxin, Choleratoxin, Adenylatcyclase aktivierendes Polypeptid, *Staphylococcus* Enterotoxin, Rizin A Kette, Conotoxin, Charybdotoxirin und Sarafotoxin, RTX-Toxine, wie ApxI, ApxII oder ApxIII, Shigatoxin aus *Shigella* Spezies, IpaH aus *Shigella* Spezies und EIEC, InvE aus *Shigella* Spezies und EIEC, AraC aus *Salmonella* Spezies, Verocytotoxin-1 aus EHEC oder VTEC, Verocytotoxin-2 aus EHEC oder VTEC, toxischer Schocksyndrom Toxin-1 aus *Staphylococcus aureus*, Ctx aus *Vibrio cholerae* und Enterotoxin aus *Clostridium perfringens*. Weitere Toxine sind SLTs, wie zum Beispiel diejenigen aus *Salmonella, Campylobacter* und anderen Bakterien, *Clostridium difficile* Toxine A und B, bakterielle Pili aus enteropathogenen *E. coli* (EPEC) und enterotoxigenen *E. coli* (ETEC) und virale Lectine, wie zum Beispiel virale Hämagglutinine. Noch weitere Toxine sind Herzglycoside (z.B. Strophantin, Cardenolide, Bufadienolide, usw.), Kalziumkanal-Blocker, Aflatoxine, Azaserine, Aminopterin, Saponine, Pertussistoxin, Choleratoxin, Butolinotoxin, usw.

Erfindungsgemäß weiter bevorzugt ist ein Verfahren, wobei die toxische Komponente ausgewählt ist aus Antibiotika, wie zum Beispiel beta-Lactam Antibiotika.

Antibiotika erreichen ihren antibakteriellen Effekt durch verschiedene Wirkmechanismen, die im allgemeinen wie folgt gruppiert werden können: (1) Mittel, die auf die bakterielle Zellwand wirken, wie zum Beispiel Bacitracin, die Cephalosporine, Cycloserine, Fosfomycin, die Penicilline, Ristocetin und Vancomycin; (2) Mittel, die die Zellmembran beeinträchtigen oder einen Detergenzeffekt ausüben, wie zum Beispiel Colistin, Novobiocin und Polymyxin; (3) Mittel, die die zellulären Mechanismen der Replikation, des Informationstransfers und der Proteinsynthese durch ihre Effekte auf Ribosomen beeinträchtigen, zum Beispiel die Aminoglycoside, die Tetracycline, Chloramphenicol, Clindamycin, Cycloheximid; Fucidin, Lincomycin, Poromycin, Rifampicin, andere Streptomycine, und die Macrolid-Antibiotika wie zum Beispiel Erythromycin und Oleandomycin; (4) Mittel, die den Nukleinsäure-Metabolismus beeinträchtigen, z. B. die Fluorchinolone, Actinomycin, Ethambutol, 5-Fluorcytosin, Griseofulvin, Rifamycin; und (5) Wirkstoffe, die den intermediären Metabolismus beeinträchtigen, wie zum Beispiel die Sulfonamide, Trimethoprim und die tuberkulostatischen Mittel Isoniazid und para-Aminosalizylsäure. Erfindungsgemäß kann die toxische Komponente somit ausgewählt sein aus β-Lactam-Antibiotika, einschließlich eines Penicillin-, eines Cephalosporin-, Imipenems- oder eines Monobactam-Antibiotikums, eines Aminoglycosid-Antibiotikums, eines Tetracyclin-Antibiotikums, eines Sulfonamid-Antibiotikums, eines Trimethoprim-Antibiotikums, Trimethoprim/Sulfamethoxazols, eines Fluorochinolon-Antibiotikums, eines Chinolon-Antibiotikums, eines Macrolid-Antibiotikums, eines Polymyxin-Antibiotikums, Vancomycin, Chloramphenicol, Rifampin, Augmentin, Nitrofurantoin, Lincomycin oder Clindamycin oder anderen Antibiotika, wie Neomycin, Hygromycin, Gentamycin, Colchicin.

Es kann auch ein Gemisch von mehreren toxischen Komponenten an einem oder verschiedenen Konjugaten verwendet werden, so daß auf mehrere gleichzeitig exprimierte bindende Moleküle gleichzeitig gescreent werden kann und auf eine spezifische Auswahl aus einem Gemisch von zu selektionierenden Zellen gescreent werden kann oder eine Selektion von eukaryontischen Zellen unter gleichzeitiger Abtötung prokaryontischer Kontaminanten erfolgen kann.

Offenbart wird weiter ein Verfahren, wobei das Effektormolekül, wie etwa der Suppressor/Aktivator, ausgewählt ist aus Gruppen, die in der Zelle eine nachweisbare Stoffwechselveränderung hervorrufen oder inhibieren, Gruppen, die die Expression eines Reportergens hervorrufen oder inhibieren und Transskriptionsfaktoren. Neben der negativen Selektion kann das erfindungsgemäße Verfahren auch im Rahmen einer positiven Selektion angewendet werden. So besteht die Möglichkeit, auf andere Signale, als der Unterscheidung zwischen lebenden/wachsenden und toten/ruhenden Zellen zu selektionieren, indem ein Suppressor/Aktivator-Konjugat verwendet wird, in dem z.B. ein Zelltyp farbig wird durch die Wechselwirkung mit dem bindenden Konjugat. Eine denkbare Wirkungskette wäre dabei ein Konjugat aus Suppressor/Aktivator-Ligand, das an das Molekül bindet. Dadurch bleibt das Konjugat inaktiv, eine intrazelluläre Stoffwechselveränderung erfolgt nicht, ein Reportergen wird nicht exprimiert oder umgekehrt wodurch sich der Phänotyp der Zelle ändert (z.B. nicht durch GFP-Protein-Expression leuchtet oder das lacZ-System exprimiert wird). Eine weitere Möglichkeit wäre die Ergänzung des Two-Hybrid-Systems durch Ligand-gekoppelte Transskriptionsfaktoren.

Der Ligand des erfindungsgemäß eingesetzten Konjugats wird in Abhängigkeit des jeweiligen zu selektionierenden spezifisch bindenden Moleküls ausgewählt. So betrifft ein Aspekt des erfindungsgemäßen Verfahrens, daß der spezifische Ligand ausgewählt ist aus Molekülen, gegen die ein spezifischer Binder selektioniert werden soll. Grundsätzlich kann der spezifische Ligand ausgewählt sein aus Molekülen mit hohem Molekulargewicht, kleinem Molekulargewicht und kovalent oder nicht kovalent miteinander verbundenen Liganden-Komplexen. Ein solcher Komplex kann aus zwei (oder mehr) Teilen oder Untereinheiten bestehen, die dann gemeinsam miteinander einen spezifischen Liganden ergeben. Verfahren zur Herstellung von solchen Liganden-Komplexen sind dem Fachmann bekannt.

Beispiele für Liganden-Moleküle sind ausgewählt aus Antikörpern, Viren, chemotherapeutischen Mitteln, Rezeptor-Agonisten und -Antagonisten, Antikörperfragmenten, Lectinen, Albuminen, Peptiden, Proteinen, Hormonen, Aminozuckern, Lipiden, Fettsäuren und Nukleinsäuren und/oder aus Molekülen mit relativ kleinem Molekulargewicht, insbesondere Peptiden, Oligonukleotiden, Sacchariden, wie Mono-, Di-, Poly- oder Oligosacchariden, Blutgruppenfaktoren, Thomsen-Friedenreich Antigen und Muzinen und Proteinen, ausgewählt aus Glycoproteinen, Hormonen, Interleukinen, Wachstumsfaktoren, Wachstumsinhibitoren, Adhäsionsmolekülen, Lektinen, Enzymen, Differenzierungsantigenen (CD-Markern), Kinasen, T-Zell-Rezeptoren, zellulären Rezeptoren, Antikörpern oder Antikörperfragmenten, katalytischen Antikörpern, MHC-Molekülen und ähnlichen, Haptenen, Toxinen, Pharmaka, Herbiziden, Fungiziden, Insektiziden, Pflanzenschutzmitteln, Toxinen, inklusive Antibiotika (nicht diejenigen für das eingesetzte Zellsystem), Dogen und Rauschgifte, Dopingmittel, Substratanaloga von Enzymen, Chemotherapeutika, Farbstoffen, Stoffwechselmetaboliten und ähnlichen ausgewählt sein. Bevorzugt ist ein Verfahren gemäß der vorliegenden Erfindung, wobei der spezifische Ligand ausgewählt ist aus Molekülen mit relativ kleinem Molekulargewicht, insbesondere Peptiden, Oligonukleotiden, Oligosacchariden, Haptenen, Toxinen, Pharmaka, Herbiziden, Fungiziden, Farbstoffen, Stoffwechselmetaboliten und ähnlichen.

Es kann auch zunächst auf eine Ligand-Kernstruktur hin selektioniert werden, die in anschließenden weiteren Screening-Runden weiter chemisch und strukturell spezifiziert oder definiert wird. Eine diesbezügliche Vorauswahl kann aufgrund von bekannten Liganden getroffen werden, die dann weiter modifiziert werden. Dazu kann unter anderem das sogenannte "Molecular Modelling" eingesetzt werden, deren Grundzüge dem Fachmann bekannt sind und unter anderem in Nagl SB. Computational function assignment for potential drug targets: from single genes to cellular systems. Curr Drug Targets. 2002 Oct;3(5):387-99; Wormald MR, Petrescu AJ, Pao YL, Glithero A, Elliott T, Dwek RA. Conformational studies of oligosaccharides and glycopeptides: complementarity of NMR, X-ray crystallography, and molecular modelling. Chem Rev. 2002 Feb;102(2):371-86. Review; Smith GR, Sternberg MJ. Prediction of protein-protein interactions by docking methods. Curr Opin Struct Biol. 2002 Feb;12(1):28-35 und Forster MJ. Molecular modelling in structural biology. Micron. 2002;33(4):365-84 beschrieben sind. Es kann auch eine Kombination oder ein Gemisch von Liganden verwendet werden, so daß auf mehrere gleichzeitig exprimierte bindende Moleküle gleichzeitig gescreent werden kann.

Das grundlegende Prinzip des Verfahrens gemäß der vorliegenden Erfindung beruht darauf, daß, wenn eine zu selektionierende Zelle oder Gewebe ein Molekül synthetisiert, das den Liganden bindet, gleichzeitig eine Neutralisation der z. B. toxischen Wirkung der toxischen Komponente der Effektormolekül-Komponente erfolgt. Das Prinzip der vorliegenden Erfindung steht somit genau im Gegensatz zu verschiedenen im Stand der Technik vorhandenen "Konjugat-Ansätzen", wie zum Beispiel der sogenannte Ultra-Potent Toxin (UPT)-Technologie, mittels der Antikörper-Toxin-Konjugate generiert werden können. Diese Konjugate sind ein viel versprechender Ansatz vor allem in der Krebsforschung: Die Antikörper können entartete Tumorzellen spezifisch erkennen, docken an diese an und setzten das Toxin frei, das schließlich die Tumorzellen abtötet.

Im weiteren Verlauf einer bevorzugten Ausführungsform der vorliegenden Erfindung wird der Effekt, wie etwa die Toxizität, des Konjugats zunächst überprüft und eine Arbeitskonzentration bestimmt, bei welcher native Zellen absterben bzw. im Wachstum inhibiert werden. Im eigentlichen Selektionsschritt werden daher nur diejenigen Zellen überleben, welche in der Lage sind, einen geeigneten Binder zu produzieren. Im Falle einer positiven Selektion, d.h. einer Suppressor/Aktivator-Komponente erfolgt die Bestimmung analog.

Gemäß einer Alternative des beschriebenen Verfahrens kann mit einer Zelle, die einen bestimmten Binder synthetisiert, aus einer Bibliothek von Effektormolekül-Liganden Konjugaten der optimale Ligand selektionert werden. Es wird somit nicht auf eine bestimmte Zelle, sondern auf einen bestimmten Liganden gescreent. Solche Ligandenbibliotheken können z. B. mittels dem Fachmann bekannter kombinatorischer Chemie hergestellt werden.

Neben den oben genannten Vorteilen ergeben sich aus der vorliegenden Erfindung noch weitere Vorteile, die dem Fachmann bei der Durchführung des Verfahrens ersichtlich werden. Beispielhaft seien hier nur folgende genannt:
- eine Resistenzentwicklung gegenüber den Konjugaten wird gegenüber herkömmlicher einfacher Antibiotika-Selektionierung erschwert, da die Konjugate als Substrate vieler Antibiotika-zersetzenden Enzyme (wie beta-Lactamasen) nicht erkannt werden sollten. Dies ermöglicht eine lange Verweildauer des Selektionsmittels in der zu selektionierenden Kultur.
- Die Konjugate erlauben eine positive (Auswahl einer ein Molekül produzierenden Zelle) oder negative (Auswahl einer ein bestimmtes Molekül nicht produzierende Zelle) Selektion in Abhängigkeit des Konjugats.
- Das erfindungsgemäße Verfahren ist breit einsetzbar, bei gleichzeitigem geringen Aufwand und simpler Methodik.
- Die Effizienz des erfindungsgemäßen Verfahrens läßt sich durch dessen Integration in ein Hoch-Durchsatz-Verfahren ("High-troughput-Environment") noch erhöhen. Solche Verfahren schließen die Verwendung von Robotern und Bio-Chips ein, und beinhalten üblicherweise die Immobilisierung mindestens eines der Bindungspartner (Zelle oder Konjugat). Solche Techniken sind zum Beispiel in Galcheva-Gargova Z, Tao J, Chapple JP, Lapan KA, Yang M. Peptide ligands in antibacterial drug discovery: use as inhibitors in target validation and target-based screening. Expert Opin Ther Targets. 2002 Aug;6(4):507-16L; Macarron R, Hertzberg RP. Design and implementation of high throughput screening assays. Methods Mol Biol. 2002;190:1-29 und Griffin TJ, Han DK, Gygi SP, Rist B, Lee H, Aebersold R, Parker KC. Toward a high-throughput approach to quantitative proteomic analysis: expression-dependent protein identification by mass spectrometry. J Am Soc Mass Spectrom. 2001 Dec;12(12):1238-46 beschrieben.

Das erfindungsgemäße Verfahren eignet sich sowohl für prokaryontische (z.B. bakterielle Zellen, wie Archaebakterien und Eubakterien, wie etwa *E. coli*) als auch für eukaryontische Zellen (z. B. Hefezellen, Pilzzellen, Insektenzellen, Algenzellen, Pflanzenzellen und Säugerzellen, insbesondere Krebszelllinien, Hybridomzellen, z.B. Hybriden aus B-Lymphozyten und möglichen Fusionspartnern, wie z.B. Hefen, Algen, Pflanzenzellen und anderen als den explizit in der Hybridomtechnik verwendeten Säugerzellen und Stammzellen, insbesondere nicht-menschlichen embryonalen Stammzellen). Es können auch Binder-produzierende Zellen verwendet wurden, die durch andere Wege dazu transformiert wurden, sich unbegrenzt zu teilen und in Kultur zu wachsen, wie etwa durch transformierende Gene, Viren, virale Vektoren, Gentransfer, Gendeletion oder Gensuppression.

Weiterhin läßt sich das erfindungsgemäße Verfahren auch auf Zellen anwenden, die in Form eines Organs und/oder Gewebes vorliegen und kann somit im Rahmen des "Tissue Engineering" verwendet werden. Vorteilhafterweise kann dabei die Selektion in vitro oder sogar in vivo durchgeführt werden. Dies hängt von dem jeweils zu selektionierenden Molekül ab. Das erfindungsgemäße Verfahren kann somit auch im Bereich der Erzeugung von transgenen Organismen, wie nicht-menschlichen Säugern oder Pflanzen angewendet werden. So besteht die Möglichkeit, daß transgene Pflanzen und Tiere, die erwünschte Eigenschaften erhalten, dadurch hergestellt werden, daß man sie hier über den dargestellten Weg selektiert (z.B. um über die Kalluskultur eine Pflanze mit Resistenzen gegen Herbizide, Insekten, Pilze, Hefen, Parasiten, usw. oder über eine Stammzellkultur Tiere mit gewünschten Eigenschaften herstellt) und z.B. im so genannten "Pharming" für die Produktion bestimmter Substanzen einsetzt. Weiterhin ist, wie bereits oben erwähnt, die Selektion von Liganden aus einer Ligandenbibliothek möglich.

Gemäß einem weiteren Aspekt der vorliegenden Erfindung werden als Moleküle Nukleinsäuren, Polysaccharide oder Proteine von den zu selektionierenden Zellen exprimiert. Diese Moleküle können alle Moleküle sein, die in der Lage sind, den Liganden des erfindungsgemäß eingesetzten Konjugats zu binden, um so die Wirkung de Effektormoleküls, wie etwa der toxischen Komponente, zu verringern oder aufzuheben.

Bevorzugt ist ein erfindungsgemäßes Verfahren, wobei die Nukleinsäuren ausgewählt sind aus Oligonukleotiden in Form von DNA oder RNA, antigenen Oligonukleotiden, Ribozymen und Aptameren; die Sacharide ausgewählt sind aus Poly- oder Oligosacchariden, wie Blutgruppenfaktoren, Thomsen-Friedenreich Antigen und Muzinen und ähnlichen, und die Proteine ausgewählt sind aus Glycoproteinen, Hormonen, Interleukinen, Wachstumsfaktoren, Wachstumsinhibitoren, Adhäsionsmolekülen, Lektinen, Albuminen, Enzymen (die an Substrat-analoge Liganden binden), Differenzierungsantigenen (CD-Markern), Kinasen, T-Zell-Rezeptoren, zellulären Rezeptoren, Antikörpern oder Antikörperfragmenten, katalytischen Antikörpern, MHC-Molekülen und andere bindenden Peptiden. Besonders bevorzugt sind Antikörperfragmente, wie scFv oder Fab.

Gemäß einem weiteren Aspekt des Verfahrens der vorliegenden Erfindung umfaßt die Expression der Moleküle eine Exkretion der Moleküle in das Medium, eine Expression in der Membran der Zelle, eine Expression im endoplasmatischen Retikulum, eine Expression im Apoplasten und/oder eine cytoplasmatische oder periplasmatische Expression. Die Art der Expression spielt solange keine Rolle, wie eine Konjugat-vermittelte Selektion der Expression erfolgen kann. Das zur Selektion verwendete Konjugat kann somit auch intrazellulär wirken. Ein von der Zelle selbst synthetisiertes Konjugat könnte ebenfalls eingesetzt werden.

Gemäß einem noch weiteren Aspekt der vorliegenden Erfindung kann die Selektion gemäß des erfindungsgemäßen Verfahrens gemeinsam mit anderen toxische Komponenten, ausgewählt aus Toxinen, wie bakteriellen Toxinen, Antibiotika, wie beta-Lactam Antibiotika und anderen Zellen abtötende oder Wachstums-inhibitorischen Substanzen erfolgen.

Gemäß einem noch weiteren Aspekt der vorliegenden Erfindung kann die Selektion gemäß des erfindungsgemäßen Verfahrens zu einer sogenannten Affinitätsreifung oder auch Mutagenese des Moleküls führen. Dabei kann ein selektierter Binder mutiert und erneut gegen steigende Konzentrationen des Konjugats selektioniert werden. Die Zelle kann dem gestiegenen Selektionsdruck durch die Produktion von quantitativ mehr oder "verbessertem" (mutiertem) Binder entgehen. Beide Varianten können weiterhin von Nutzen sein.

Die Anwendung des offenbarten Verfahrens ist nicht auf Zellen oder Gewebe beschränkt, in denen das zu selektionierende Molekül rekombinant exprimiert wird. Vielmehr könne auch natürlicherweise (oder "endogen") exprimierte Moleküle selektioniert werden. Diese können zum Beispiel von Krebszellen an der Oberfläche präsentierte Antigene, Rezeptoren, Apoptose-spezifische und weitere exprimierte Moleküle sein. Auch Peptid-Antibiotika oder Nukleinsäuren, gehören dazu.

Wird aus großen, zufällig generierten Bibliotheken selektiert, werden unter Umständen in einigen Ansätzen auch neben spezifischen Bindern des Liganden auch Binder des Toxinanteils bzw. der Übergangsstrukturen selektiert. Eine Charakterisierung der Spezifität der Bindungsmoleküle der überlebenden und/oder supprimierten/aktivierten Zellen und ihrer produzierten Binder ist deshalb im Anschluß erforderlich, wobei auch die Auffiridung solcher nicht Liganden-spezifischen Bindepartner für die weitere Verwendung der Ergebnisse des Selektionsverfahrens von Vorteil sein kann, da die Konjugate geeignet angepaßt werden können.

Eine bevorzugte Anwendung des Verfahrens der vorliegenden Erfindung ist z. B. die Selektion von spezifischen Antikörpern, die z. B. in der Medizin, Umwelttechnik, Diagnostik etc., eingesetzt werden können und die Selektion von spezifischen Bindern in der Pharmabranche für Wirkstoffentwicklung, die dann ebenfalls in der Medizin zum Einsatz kommen können. Weiterhin ist es möglich, mit Hilfe des erfindungsgemäßen Verfahrens über den oben beschriebenen Weg der Affinitätsreifung von Bindern zu Katalyten, z.B. katalytischen Antikörpern, zu gelangen.

Die Erfindung ermöglicht das leichtere und am Ende billigere Auffinden bindender Moleküle. Sie ermöglicht darüber hinaus im Vergleich zu den bisher üblichen Screeningmethoden den Zugriff auf sehr seltene Binder, da aus großen Mengen potentieller Binder selektiert werden kann.

Ein weiterer Aspekt der vorliegenden Erfindung betrifft die Verwendung eines erfindungsgemäßen Verfahrens zum "Tissue Engineering" in vivo oder in vitro. Der Begriff Tissue Engineering bedeutet dabei die Anwendung des erfindungsgemäßen Verfahrens im Rahmen der Erzeugung eines mit bestimmten Eigenschaften versehenen Gewebes, d.h. mehr als einer einzelnen Zelle. Solche Ansätze werden in vielen Bereichen insbesondere der Transplantationsmedizin verfolgt und sind unter anderem in folgenden Publikationen beschrieben: Young BH, Peng H, Huard J. Muscle-based gene therapy and tissue engineering to improve bone healing. Clin Orthop. 2002 Oct;(403 Suppl):S243-51; Shimmura S, Tsubota K. Ocular surface reconstruction update. Curr Opin Ophthalmol. 2002 Aug;13(4):213-9; Murota SI. Opening to the age of regenerative medicine--the mechanism of angiogenesis Kokubyo Gakkai Zasshi. 2002 Jun;69(2):81-8 und Saltzman WM, Olbricht WL. Building drug delivery into tissue engineering. Nat Rev Drug Discov. 2002 Mar;1(3):177-86.

Ein noch weiterer Aspekt betrifft eine Molekül-exprimierende Zelle oder ein Gewebe oder nicht-menschliches Tier oder eine Pflanze, die Zellen enthält, die mittels eines erfindungsgemäßen Verfahrens wie oben selektioniert wurde(n). Dabei kann diese Zelle eine Bakterienzelle, Hefezelle, Pilzzelle, Insektenzelle, Algenzelle, Pflanzenzelle oder Säugerzelle, insbesondere Hybridomzelle oder Stammzelle, insbesondere Stammzelle, insbesondere nichtmenschliche embryonale Stammzelle sein, die entweder das selektionierte Molekül rekombinant oder endogen exprimiert.

Die für die erfindungsgemäßen Verfahren verwendeten Materialien sind für die Herstellung eines Kits (Testsatz) ideal geeignet. Daher betrifft die Erfindung weiterhin ein Kit, umfassend Materialien zur Durchführung des erfindungsgemäßen Verfahrens, zusammen mit weiteren Hilfs- und Zusatzstoffen. Zum Beispiel stellt die vorliegenden Erfindung ein kompartimentalisiertes Kit zur Verfügung, das einen oder mehrere Behälter umfaßt, die a) einen ersten Behälter mit einem Konjugat zur Durchführung des Screenings auf ein zu exprimierendes Molekül und b) einen oder mehrere Behälter, die geeignete Medien und Zellen für das Verfahren enthalten können. Im erfindungsgemäßen Kit könnte auch ein kopplungsfähiges Toxin zusammen mit einer Binder-produzierenden Zellbibliothek zur Durchführung des Verfahrens der vorliegenden Erfindung angeboten werden.

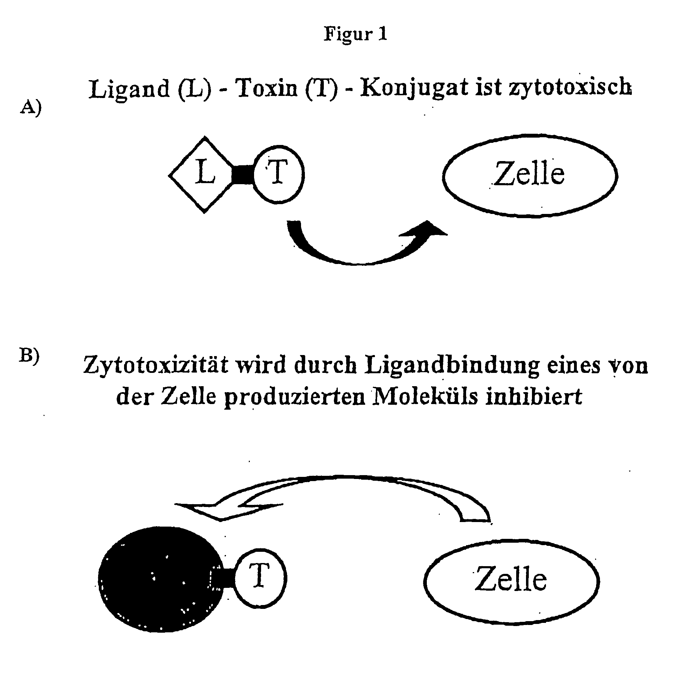

Die Erfindung soll nun in den folgenden Beispielen unter Bezug auf die beigefügte Zeichnung näher beschrieben werden, ohne jedoch auf diese beschränkt zu werden. Dabei zeigt
Figur 1: Den schematischen Ablauf des erfindungsgemäßen Verfahrens am Beispiel der toxischen Wirkung eines Ampicillin-Fluorescein-Konjugats auf *E. coli* (Figur 1 A) und der toxischen Wirkung eines Ampicillin-Fluorescein-Konjugats auf Einketten-Antikörper-Fragment exprimierende *E. coli* (Figur 1 B). L = Ligand/Binder; T=toxische Komponente

### Beispiel 1: Toxische Wirkung eines Ampicillin-Fluorescein-Konjugats auf E. coli (Figur 1 A)

Bei den in diesem Modellexperiment verwendeten Zellen handelte es sich um das Bakterium *E. coli*. Als Toxin-Ligand-Konjugat wurde ein Konjugat aus Ampicillin (Toxin) und Fluorescein (Ligand) hergestellt und anschließend verwendet. Die Kopplung erfolgte hierbei über die Aminogruppe des Ampicillins.

Das Ampicillin-Fluorescein-Konjugat zeigte wie Ampicillin (Positivkontrolle) eine Toxizität für *E. coli*. Externe Gaben eines Anti-Fluorescein-Antikörpers (B13DE1) bewirken das Überleben der Zellen, was die Beseitigung der toxischen Wirkung des Ampicillin-Anteils im Konjugat bestätigte.

### Beispiel 2: Toxische Wirkung eines Ampicillin-Fluorescein-Konjugats auf Einketten-Antikörper-Fragment exprimierende E. coli (Figur 1 B)

In diesem zweiten Experiment kamen *E. coli* zum Einsatz, die dahingehend gentechnisch verändert wurden, daß sie ein Einketten-Antikörper-Fragment (single-chain fragment, scFv) exprimierten, das den Liganden Fluorescein bindet.

Die Expression dieses gegen den Liganden des Toxin-Ligand-Konjugat aus Beispiel 1 gerichteten Einketten-Antikörper-Fragmentes bewirkte, ebenso wie die externen Antikörpergaben wie im ersten Experiment, ein Überleben der Zellen.

## Patentansprüche

1. Verfahren zur Selektion von Zellen, die spezifisch bindende Moleküle rekombinant exprimieren, umfassend
a) zur Verfügung stellen von Zellen, die potentiell mindestens ein spezifisch bindendes Molekül rekombinant exprimieren,
b) in Kontakt bringen der Zellen aus Schritt a) mit einem Konjugat, umfassend mindestens i) eine Effektormolekül-Komponente und ii) einen spezifischen Liganden für das mindestens eine spezifisch bindende Molekül, und
c) Selektion der Zellen, die das mindestens eine spezifisch bindende Molekül rekombinant exprimieren, indem das spezifisch bindende Molekül das Effektormolekül über die Bindung an den Liganden inaktiviert oder neutralisiert, und
wobei das Effektormolekül ein Zellen abtötendes Toxin ist.

2. Verfahren nach Anspruch 1, in dem Schritt b) umfaßt
b') Synthetisieren eines Konjugats aus mindestens einem Effektormolekül und dem mindestens einen spezifischen Liganden für das mindestens eine spezifisch bindende Molekül,
b") Überprüfen der Aktivität des Konjugats an Zellen, die keinen spezifischen Liganden exprimieren und Ermitteln geeigneter Selektionsbedingungen, und
b"') in Kontakt bringen der Zellen aus Schritt a) mit einer geeigneten Konzentration des Konjugats.

3. Verfahren nach Anspruch 1 oder 2, wobei die Zellen ausgewählt sind aus pro- oder eukaryontischen Zellen.

4. Verfahren nach Anspruch 3, wobei die Zellen ausgewählt sind aus der Gruppe von Zellen umfassend Bakterienzellen, Archaebakterien, Hefezellen, Pilzzellen, Insektenzellen, Algenzellen, Pflanzenzellen und Säugerzellen, insbesondere Hybridomzellen, z.B. Hybriden aus B-Lymphozyten und möglichen Fusionspartnern und Stammzellen, insbesondere nicht-menschlichen embryonalen Stammzellen.

5. Verfahren nach einem der Ansprüche 1 bis 4, wobei die Zellen in Form eines Organs und/oder Gewebes vorliegen.

6. Verfahren nach einem der Ansprüche 1 bis 5, wobei die Selektion *in vitro* oder *in vivo* durchgeführt wird.

7. Verfahren nach einem der Ansprüche 1 bis 6, wobei als Moleküle Nukleinsäuren, Saccharide, Peptide oder Proteine rekombinant exprimiert werden.

8. Verfahren nach Anspruch 7, wobei die Nukleinsäuren ausgewählt sind aus Oligonukleotiden in Form von DNA oder RNA, antisense Oligonukleotiden, Protein bindenden Oligonukleotiden, Ribozymen und Aptameren; die Sacharide ausgewählt sind aus Poly- oder Oligosacchariden, wie Blutgruppenfaktoren, Thomsen-Friedenreich Antigen und Muzinen und ähnlichen, und die Proteine ausgewählt sind aus Glycoproteinen, Hormonen, Interleukinen, Wachstumsfaktoren, Wachstumsinhibitoren, Adhäsionsmolekülen, Lektinen, Enzymen, Differenzierungsantigenen (CD-Markern), Kinasen, T-Zell-Rezeptoren, zellulären Rezeptoren, Antikörpern oder Antikörperfragmenten, katalytischen Antikörpern, MHC-Molekülen, anderen bindenden Peptiden und ähnlichen.

9. Verfahren nach einem der Ansprüche 1 bis 8, wobei die Expression der Moleküle eine Exkretion der Moleküle in das Medium, eine Expression in der Membran der Zelle, eine Expression im endoplasmatischen Retikulum, eine Expression im Apoplasten und/oder eine cytoplasmatische oder periplasmatische Expression umfaßt.

10. Verfahren nach einem der Ansprüche 1 bis 9, wobei das Toxin ausgewählt ist aus bakteriellen Toxinen, Antibiotika, Herzglycosiden und Kalziumkanal-Blockern.

11. Verfahren nach Anspruch 10, wobei das Toxin ausgewählt ist aus β-Lactam-Antibiotika, einschließlich eines Penicillin-, eines Cephalosporin-, Imipenems- oder eines Monobactam-Antibiotikums, eines Aminoglycosid-Antibiotikums, eines Tetracyclin-Antibiotikums, eines Sulfonamid-Antibiotikums, eines Trimethoprim-Antibiotikums, Trimethoprim/Sulfamethoxazols, eines Fluorochinolon-Antibiotikums, eines Chinolon-Antibiotikums, eines Macrolid-Antibiotikums, eines Polymyxin-Antibiotikums, Vancomycin, Chloramphenicol, Rifampin, Augmentin, Nitrofurantoin, Lincomycin oder Clindamycin oder anderen Antibiotika, wie Neomycin, Hygromycin, Gentamycin, Colchicin.

12. Verfahren nach einem der Ansprüche 1 bis 11, wobei der spezifische Ligand ausgewählt ist aus Molekülen, gegen die ein spezifischer Binder selektioniert werden soll.

13. Verfahren nach einem der Ansprüche 1 bis 12, wobei der spezifische Ligand ausgewählt ist aus Molekülen mit hohem Molekulargewicht, kleinem Molekulargewicht und kovalent oder nicht kovalent miteinander verbundenen Liganden-Komplexen.

14. Verfahren nach einem der Ansprüche 1 bis 13, wobei der spezifische Ligand ausgewählt ist aus Molekülen mit relativ kleinem Molekulargewicht, insbesondere Peptiden, Oligonukleotiden, Oligosacchariden, Haptenen, Toxinen, Pharmaka, Herbiziden, Fungiziden, Farbstoffen, Stoffwechselmetaboliten und ähnlichen.

15. Verfahren nach einem der Ansprüche 1 bis 14, wobei das Effektormolekül und der Ligand des Konjugats kovalent oder nicht kovalent miteinander verbunden sind.

16. Verfahren nach einem der Ansprüche 1 bis 15, wobei das Konjugat weiterhin einen kovalenten oder nicht kovalenten Linker zwischen dem Toxin und dem Liganden aufweist.

17. Verfahren nach einem der Ansprüche 1 bis 16, wobei die Selektion gemeinsam mit anderen toxische Komponente ausgewählt aus Toxinen, wie bakteriellen Toxinen, Antibiotika, wie beta-Lactam Antibiotika und anderen Zellen abtötenden Substanzen erfolgt.

18. Verfahren nach einem der Ansprüche 1 bis 17, wobei Zellen selektiert werden, die das Ligand-Molekül in höherer Konzentration oder ein höher-affines Ligand-Molekül synthetisieren.

19. Verwendung eines Verfahrens nach einem der Ansprüche 1 bis 18 zum "Tissue Engineering" *in vivo* oder *in vitro* oder zur Selektion eines optimalen Effektormolekül-Ligand Konjugats aus einer Effektormolekül-Ligand Konjugat-Bibliothek.

20. Verwendung eines Kits, das Kit umfassend einen oder mehrere Behälter enthaltend Materialien zur Durchführung des Verfahrens nach einem der Ansprüche 1 bis 18, umfassend a) einen ersten Behälter mit einem kopplungsfähigen Toxin, b) einen zweiten Behälter mit einer Binder-produzierenden Zellbibliothek und c) einen oder mehrere Behälter enthaltend geeignete Medien und Zellen für das Verfahren.

## Claims

1. A method for selecting cells that recombinantly express specifically binding molecules, comprising
a) providing of cells that potentially recombinantly express at least one specifically binding molecule,
b) contacting the cells from step a) with a conjugate, comprising at least i) one effector molecule component and ii) one specific ligand for the at least one specifically binding molecule, and
c) selecting the cells that recombinantly express the at least one specifically binding molecule through inactivating or neutralizing the effector molecule through binding of the specifically binding molecule to the ligand, and
wherein the effector molecule is a cytotoxic toxin.

2. The method according to claim 1, wherein step b) comprises
b') synthesizing of a conjugate of at least one effector molecule and the at least one specific ligand for the at least one specifically binding molecule,
b") testing of the activity of the conjugate on cells that do not express a specific ligand, and detecting of suitable conditions for selection, and
b"') contacting the cells from step a) with a suitable concentration of the conjugate.

3. The method according to claim 1 or 2, wherein the cells are selected from pro- or eukaryotic cells.

4. The method according to claim 3, wherein the cells are selected from the group of cells comprising bacterial cells, archaebacteria, yeast cells, fungal cells, insect cells, algal cells, plant cells, and mammalian cells, in particular hybridoma cells, e.g. hybrids of B-lymphocytes and possible fusion partners and stem cells, in particular non-human embryonic stem cells.

5. The method according to any of claims 1 to 4, wherein the cells are present in the form of an organ and/or tissue.

6. The method according to any of claims 1 to 5, wherein the selection is performed in *vitro* or *in vivo.*

7. The method according to any of claims 1 to 6, wherein nucleic acids, saccharides, peptides or proteins are recombinantly expressed as molecules.

8. The method according to claim 7, wherein the nucleic acids are selected from oligonucleotides in the form of DNA or RNA, antisense oligonucleotides, protein binding oligonucleotides, ribozymes and aptamers; the saccharides are selected from poly- or oligosaccharides, such as blood type factors, Thomsen-Friedenreich antigen and mucines and the like, and the proteins are selected from glycoproteins, hormones, interleukins, growth factors, growth inhibitors, adhesion molecules, lectins, enzymes, differentiation antigens (CD-markers), kinases, T-cell-receptors, cellular receptors, antibodies or fragments of antibodies, catalytic antibodies, MHC-molecules, other binding peptides, and the like.

9. The method according to any of claims 1 to 8, wherein the expression of the molecules comprises an excretion of the molecules into the medium, an expression in the membrane of the cell, an expression in the endoplasmatic reticulum, an expression in apoplasts and/or a cytoplasmatic or periplasmatic expression.

10. The method according to any of claims 1 to 9, wherein the toxin is selected from bacterial toxins, antibiotics, heart glycosides and calcium channel-blockers.

11. The method according to claim 10, wherein the toxin is selected from β-lactam-antibiotics, including a penicillin-, a cephalosporin-, imipenem- or a monobactam-antibiotic, an aminoglycoside-antibiotic, a tetracycline-antibiotic, a sulfonamide-antibiotic, a trimethoprime-antibiotic, trimethoprime/sulfamethoxazole, a fluorochinolone-antibiotic, a chinolone-antibiotic, a macrolide-antibiotic, a polymyxine-antibiotic, vancomycin, chloramphenicol, rifampin, augmentin, nitrofurantoin, lincomycin or clindamycin or other antibiotics, such as neomycin, hygromycin, gentamycin, colchicine.

12. The method according to any of claims 1 to 11, wherein the specific ligand is selected from molecules against which a specific binding agent shall be selected.

13. The method according to any of claims 1 to 12, wherein the specific ligand is selected from molecules with high molecular weight, low molecular weight and ligand-complexes that are bound to each other covalently or non-covalently.

14. The method according to any of claims 1 to 13, wherein the specific ligand is selected from molecules with relatively low molecular weight, in particular peptides, oligonucleotides, oligosaccharides, haptenes, toxins, pharmaceutics, herbicides, fungicides, dyes, metabolites of the metabolism, and the like.

15. The method according to any of claims 1 to 14, wherein the effector molecule and the ligand of the conjugate are bound to each other covalently or non-covalently.

16. The method according to any of claims 1 to 15, wherein the conjugate furthermore has a covalent or non-covalent linker between the toxin and the ligand.

17. The method according to any of claims 1 to 16, wherein the selection takes place together with other toxic components selected from toxins, such as bacterial toxins, antibiotics, such as beta-lactam antibiotics and other cytotoxic substances.

18. The method according to any of claims 1 to 17, wherein cells are selected that synthesize the ligand-molecule in a higher concentration or synthesize a higher-affine ligand-molecule.

19. Use of the method according to any of claims 1 to 18 for "tissue engineering" *in vivo* or *in vitro* or for the selection of an optimal effector molecule-ligand conjugate from an effector molecule-ligand conjugate-library.

20. Use of a kit, said kit comprising one or more container containing materials for performing the method according to any of claims 1 to 18, comprising a) a first container with a toxin suitable for coupling, b) a second container with a binding agent-producing cell library and c) one or more container containing suitable media and cells for said method.

## Revendications

1. Procédé en vue de la sélection de cellules, qui expriment d'une manière recombinante les molécules de liaison spécifique, comprenant
a) la mise à disposition de cellules, qui expriment d'une manière recombinante la potentiellement au moins une molécule de liaison spécifique,
b) la mise en contact des cellules provenant de l'étape a) avec un conjugué, comprenant au moins i) un composant de molécule effectrice et ii) un liant spécifique pour la au moins une molécule de liaison spécifique, et
c) la sélection des cellules, qui expriment d'une manière recombinante la au moins une molécule de liaison spécifique, en ce que la molécule de liaison spécifique inactive ou neutralise la molécule effectrice par l'intermédiaire de la liaison aux ligands, et
sachant que la molécule effectrice est une toxine détruisant les cellules.

2. Procédé selon la revendication 1, dans lequel l'étape b) comprend
b') la synthèse d'un conjugué d'au moins une molécule effectrice et du au moins un ligand spécifique pour la au moins une molécule de liaison spécifique,
b") le contrôle de l'activité du conjugué sur les cellules qui n'expriment aucun ligand spécifique et la détermination de conditions de sélection appropriées et
b"') la mise en contact des cellules provenant de l'étape a) avec une concentration appropriée du conjugué.

3. Procédé selon la revendication 1 ou 2, sachant que les cellules sont sélectionnées parmi des cellules procaryotes ou eucaryotes.

4. Procédé selon la revendication 3, sachant que les cellules sont sélectionnées parmi le groupe de cellules comprenant les cellules bactériennes, les Archaebactéries, les cellules de levure, les cellules de champignons, les cellules d'insectes, les cellules d'algues, les cellules de plantes et les cellules de mammifères, en particulier les cellules d'hybridome, par exemple des hybrides de lymphocytes B et de partenaires de fusion possibles et de cellules souches, en particulier de cellules souches embryonnaires non humaines.

5. Procédé selon l'une quelconque des revendications 1 à 4, sachant que les cellules sont présentes sous la forme d'un organe et/ou d'un tissu.

6. Procédé selon l'une quelconque des revendications 1 à 5, sachant que la sélection est effectuée *in vitro* ou *in vivo.*

7. Procédé selon l'une quelconque des revendications 1 à 6, sachant que l'on exprime d'une manière recombinante, en tant que molécules, des acides nucléiques, des saccharides, des peptides ou des protéines.

8. Procédé selon la revendication 7, sachant que les acides nucléiques sont sélectionnés parmi les oligonucléotides sous la forme d'ADN ou d'ARN, d'oligonucléotides antisens, d'oligonucléotides de liaison de protéines, de ribosomes et d'aptamères ; les saccharides sont sélectionnés parmi les poly ou les oligo saccharides, comme les facteurs de groupe sanguin, les antigènes Thomsen-Friedenreich et les mucines et similaires, et les protéines sont sélectionnées parmi les glycoprotéines, les hormones, les interleucines, les facteurs de croissance, les inhibiteurs de croissance, les molécules d'adhésion, les lectines, les enzymes, les antigènes de différentiation (marqueurs CD), les kinases, les récepteurs de cellules T, les récepteurs cellulaires, les anticorps ou les fragments d'anticorps, les anticorps catalytiques, les molécules CMH, d'autres peptides de liaison et similaires.

9. Procédé selon l'une quelconque des revendications 1 à 8, sachant que l'expression de la molécule comprend une excrétion de la molécule dans le milieu, une expression dans la membrane de la cellule, une expression dans le réticulum endoplasmique, une expression dans l'apoplaste et/ou une expression cytoplasmique ou périplasmique.

10. Procédé selon l'une quelconque des revendications 1 à 9, sachant que la toxine est sélectionnée parmi les toxines bactériennes, les antibiotiques, les glucosides cardiaques, les bloqueurs du canal de migration du calcium.

11. Procédé selon la revendication 10, sachant que la toxine est sélectionnée parmi les antibiotiques β-lactame, y compris un antibiotique pénicilline, céphalosporine, imipenem ou monobactame, un antibiotique aminoglycoside, un antibiotique tétracycline, un antibiotique sulfonamide, un antibiotique triméthoprim, un antibiotique triméthoprim/sulfaméthoxazol, un antibiotique fluoroquinolone, un antibiotique quinolone, un antibiotique macrolide, un antibiotique polymyxine, la vancomycine, le chloramphénicol, la rifampine, l'augmentine, la nitrofurantoïne, la lincomycine ou la clindamycine ou d'autres antibiotiques, comme la néomycine, l'hygromycine, la gentamycine, la colchicine.

12. Procédé selon l'une quelconque des revendications 1 à 11, sachant que le ligand spécifique est sélectionné parmi des molécules, contre lesquelles un liant spécifique doit être sélectionné.

13. Procédé selon l'une quelconque des revendications 1 à 12, sachant que le ligand spécifique est sélectionné parmi des molécules à poids moléculaire élevé, à poids moléculaire faible ou parmi des complexes à ligands liés entre eux d'une manière covalente ou non covalente.

14. Procédé selon l'une quelconque des revendications 1 à 13, sachant que le ligand spécifique est sélectionné parmi des molécules à poids moléculaire relativement faible, en particulier des peptides, des oligo nucléotides, des oligo saccharides, des haptènes, des toxines, des produits pharmaceutiques, des herbicides, des fongicides, des colorants, des métabolites et similaires.

15. Procédé selon l'une quelconque des revendications 1 à 14, sachant que la molécule effectrice et le ligand du conjugué sont liés l'un à l'autre d'une manière covalente ou non covalente.

16. Procédé selon l'une quelconque des revendications 1 à 15, sachant que le conjugué présente encore un groupe de liaison de nature covalente ou non covalente entre la toxine et le ligand.

17. Procédé selon l'une quelconque des revendications 1 à 16, sachant que la sélection se fait conjointement à d'autres composants toxiques sélectionnés parmi les toxines, comme les toxines bactériennes, les antibiotiques, comme les antibiotiques β-lactame ou d'autres substances détruisant les cellules.

18. Procédé selon l'une quelconque des revendications 1 à 17, sachant que l'on sélectionne des cellules qui synthétisent la molécule de ligand à raison d'une concentration plus élevée ou une molécule de ligand fortement affine.

19. Utilisation d'un procédé selon l'une quelconque des revendications 1 à 18 en vue d'une procédure de "Tissue Engineering" *in vivo* ou *in vitro* ou en vue de la sélection d'un conjugué molécule effectrice-ligand optimal provenant d'une bibliothèque conjugué ligand molécule effectrice.

20. Utilisation d'un kit, sachant que le kit comprend un ou plusieurs récipients renfermant des matériels en vue de l'exécution du procédé selon l'une quelconque des revendications 1 à 18, incluant a) un premier récipient avec une toxine capable de couplage, b) un second récipient avec une bibliothèque de cellules produisant des liants et c) un ou plusieurs récipients contenant des milieux et des cellules appropriés pour le procédé.
